# EUROPEAN PATENT SPECIFICATION

(11) **EP 2 987 800 B1**
(45) Date of publication and mention of the grant of the patent: **07.03.2018**
(21) Application number: 14785187.7
(22) Date of filing: 01.04.2014
(51) Int. Cl.: C07K 1/18, C07K 1/34, C07K 1/36, C07K 14/79

(54) **METHOD FOR SEPARATING AND PURIFYING RECOMBINED HUMAN LACTOFERRIN FROM RICE SEEDS**
VERFAHREN ZUR TRENNUNG UND REINIGUNG VON REKOMBINANTEM MENSCHLICHEM LACTOFERRIN AUS REISSAMEN
PROCÉDÉ DE SÉPARATION ET DE PURIFICATION DE LACTOFERRINE HUMAINE RECOMBINÉE À PARTIR DE GRAINS DE RIZ

(30) Priority: 16.04.2013 CN 201310131488
(43) Date of publication of application: 24.02.2016
(73) Proprietor: WUHAN HEALTHGEN BIOTECHNOLOGY CORP, East Lake High-Tech Development Zone Wuhan, Hubei 430079 (CN)
(72) Inventor: YANG, Daichang, Wuhan Hubei 430079 (CN); SHI, Jingni, Wuhan Hubei 430079 (CN); OU, Jiquan, Wuhan Hubei 430079 (CN)
(74) Representative: Høiberg P/S
(86) International application number: PCT/CN2014/074539
(87) International publication number: WO 2014/169760

(56) References cited:
- WO-A1-2005/017168
- CN-A- 1 663 961
- CN-A- 1 824 674
- ZHANG D ET AL: "Expression, purification, and characterization of recombinant human transferrin from rice (Oryza sativa L.)", PROTEIN EXPRESSION AND PURIFICATION, ACADEMIC PRESS, SAN DIEGO, CA, vol. 74, no. 1, November 2010 (2010-11), pages 69-79, XP027226638, ISSN: 1046-5928, DOI: 10.1016/J.PEP.2010.04.019 [retrieved on 2010-05-04]
- KAZUHITO FUJIYAMA ET AL: "N-Linked Glycan Structures of Human Lactoferrin Produced by Transgenic Rice", BIOSCIENCE, BIOTECHNOLOGY, AND BIOCHEMISTRY, vol. 68, no. 12, January 2004 (2004-01), pages 2565-2570, XP55018784, ISSN: 0916-8451, DOI: 10.1271/bbb.68.2565
- DIAH RACHMAWATI ET AL: "Production and Characterization of Recombinant Human Lactoferrin in Transgenic Javanica Rice", BREEDING SCIENCE, vol. 55, no. 2, January 2005 (2005-01), pages 213-222, XP55018783, ISSN: 1344-7610, DOI: 10.1270/jsbbs.55.213
- WU JINGHUAN ET AL: "[Isolation and purification of recombinant human lactoferrin (rhLF) from transgenic rice and its antibacterial activities].", WEI SHENG YAN JIU = JOURNAL OF HYGIENE RESEARCH MAY 2013, vol. 42, no. 3, May 2013 (2013-05), pages 399-404, XP008182185, ISSN: 1000-8020
- YAN, QINGQUAN ET AL.: 'Optimization of extraction conditions of Lactoferrin by response surface methodology' FOOD SCIENCE AND TECHNOLOGY vol. 36, no. 5, 20 May 2011, pages 173 - 176, XP008181378
- WANG, XUEWAN. ET AL.: 'Purification of lactoferrin from defatted milk with SPEC 70 SLS ion-exchange resin' CHEMICAL ENGINEERING ( CHINA vol. 38, 15 October 2010, pages 219 - 222, XP008181377
- GE HEALTHCARE: 'Instructions 71-5009-3-64AE Ion exchange chromatography' 31 October 2007, pages 1 - 24, XP008181392
- GE HEALTHCARE.: 'Instructions 71-5009-3-64 AE Ion exchange chromatography' 31 October 2007, pages 1 - 24

## Description

### Field of the Invention

The present invention belongs to the field of biotechnology, and particularly relates to a method for separating and purifying recombinant human lactoferrin.

### Background of the Invention

Lactoferrin (LF) is an iron binding protein that is widely present in milk and various other tissues, which is secreted by mammals, has many physiological functions such as antibacterial, anti-inflammatory and immunity enhancement. Researches in vitro test and animal experiments have shown that lactoferrin has preventive effect on intestinal microbial infection, such as wheel rotavirus, Giardia, Shigella, Salmonella infection. The inhibitory effect of lactoferrin on intestinal pathogenic bacteria is achieved mainly through two mechanisms: inhibiting the growth of bacteria; destroying the functions of virulence factor on the surface of the bacteria and therefore weakening the capacity of the virus to adhere to or invade mammalian cells. Researches show that the addition of lactoferrin in infant formula food is of great significance, and it can not only meet the needs of infant growth and development, but also benefits baby's health. The developed countries in the world like New Zealand, Switzerland, Australia, the Netherlands, have the best quality milk source of the whole world, most of the high-end infant formula milk powder of these countries contains lactoferrin, which is an important ingredient in human milk.

Due to the limitation of milk source, the supply of lactoferrin cannot meet the market demand, it is necessary to find alternative ways to obtain lactoferrin. Therefore, many researchers have expressed lactoferrin in a variety of different expression systems to obtain recombinant lactoferrin, for example in transgenic rice plants (Fujiyama, Biosci. Biotechnol. Biochem. 68 (12), 2565-2570, 2004). However, the problems such as low yield, and great difficult in purification still exist. The present applicant is dedicated to studying rice expression system and has successfully expressed recombinant human lactoferrin in rice seeds.

### Summary of the Invention

It is an object of the present invention to provide a chromatography method for separating and purifying recombinant human lactoferrin from transgenic rice seeds. The chromatography method comprises the following steps of:
1) extracting recombinant human lactoferrin from transgenic rice seeds, to obtain crude extract containing recombinant human lactoferrin;
2) subjecting the crude extract containing recombinant human lactoferrin to cation exchange chromatography to perform primary purification, to obtain an elution fraction containing recombinant human lactoferrin; wherein, the cation exchange chromatography is performed on a weak cation exchange resin, the chromatography step comprises:
   2A) equilibrating the chromatography column with 5-15 times column volume of buffer containing 10-25mM Tris, 10-25mM NaAC, 0-250mM NaCl, pH 6.0-7.5, at a flow rate of 50-200cm/h;
   2B) using the crude extract of step 1) as a loading sample, wherein the sample having a conductance of 2-25ms/cm, and a pH of 6.0-7.5; washing impurities with an impurity-washing buffer, the impurity-washing buffer containing 3-10mM NaH₂PO₄, 3-10mM Na₂HPO₄, 200-300mM NaCl, pH 6.5-8.0, and the elution flow rate being 50-200cm/h;
   2C) eluting lactoferrin with an elution buffer containing 3-10mM NaH₂PO₄, 3-10mM Na₂HPO₄, 400-650mM NaCl, pH 6.5-8.0 at a flow rate of 50-200cm/h, to obtain an elution fraction;
3) collecting the elution fraction of step 2), to obtain the solution containing recombinant human lactoferrin.

Preferably, the weak cation exchange resin of step 2) is CM sepharose FF.

The chromatography method of the present invention further comprises the following chromatography resin regeneration steps of:
a) washing the column with 3-10 times column volume of high salt solution containing 1-2M NaCl, pH 6.5-10.0 at a flow rate of 50-200cm/h until UV280 has no obvious change;
b) washing the column with 3-10 times column volume of buffer containing 5-25mM sodium citrate, pH 2.0-4.0 at a flow rate of 50-200cm/h;
c) washing the column with 2-5 times column volume of 0.5-1.0M NaOH solution at a flow rate of 30-100cm/h;
d) washing the column with 8-15 times column volume of ultrapure water to a pH of less than 10.0, keeping the resin in 20% ethanol solution.

Specifically, according to the chromatography method of the invention, wherein the extraction of recombinant human lactoferrin of step 1) comprises the following steps of:
(1) using transgenic rice seeds containing recombinant human lactoferrin as raw material, hulling the rice grains into brown rice and grinding it into rice powder with a fineness of 80-100 mesh;
(2) mixing the rice powder and extraction buffer at a weight/volume ratio of 1:5-1:10 at room temperature for 0.5-2h, to obtain a mixture; the extraction buffer containing 10-25mM Tris, 10-25mM NaAC, 0-250mM NaCl, pH 6.0-7.5;
(3) filtrating the mixture, to obtain crude extract containing recombinant human lactoferrin.

According to the chromatography method of the invention, wherein the chromatography step of step 2) comprises:
2a) equilibrating the chromatography column with 10 times column volume of equilibrium buffer containing 25mM Tris, 25mM NaAC, 200mM NaCl, pH 6.5, at a flow rate of 150cm/h;
2b) using the crude extract of step 1) as a loading sample, wherein the sample having a conductance of 22.5ms/cm, and a pH of 6.5; washing the impurities with an impurity-washing buffer, the impurity-washing buffer containing 4mM NaH₂PO₄, 6mM Na₂HPO₄, 300mM NaCl, pH7.5 at a flow rate of 150cm/h;
2c) eluting lactoferrin with an elution buffer, the elution buffer containing 4mM NaH₂PO₄, 6mM Na₂HPO₄, 600mM NaCl, pH7.5 at a flow rate of 50-200cm/h, to collect the elution fraction containing recombinant human lactoferrin;

It is another object of the present disclosure to provide an extraction method for preparing recombinant human lactoferrin from transgenic rice seeds, sequentially comprising the following steps of :
(1) using transgenic rice seeds containing recombinant human lactoferrin as raw material, hulling the rice grains into brown rice and grinding it into rice powder with a fineness of 80-100 mesh;
(2) mixing the rice powder and extraction buffer at a weight/volume ratio of 1:5-1:10 at room temperature for 0.5-2h, to obtain a mixture; the extraction buffer containing 10-25mM Tris, 10-25mM NaAC, 0-250mM NaCl, pH 6.0-7.5;
(3) filtrating the mixture, to obtain crude extract containing recombinant human lactoferrin.

### Description of Drawings

Fig.1 shows an electrophoresis image of cation exchange chromatography on CM sepharose Fast Flow;
   wherein, M: molecular mark, S: loading sample, FT: flow-through peak, Elu: rLF elution peak, Wash: impurity-washing peak.
Fig. 2 shows an electrophoresis image of cation exchange chromatography on UNO Sphere S;
   wherein, S: loading sample, FT: flow-through peak, CIP: clean in place, 20%, 40%, 60%, 100% are percentage ratios of 1.5M NaCl, respectively.
Fig. 3 shows an electrophoresis image of determination of actual load capacity of CM sepharose Fast Flow;
   wherein, M: molecular mark, Elu: rLF elution peak, Wash: impurity-washing peak, 100, 150, 200, 250, 300, 350, 400, 430, 450 are flow-through volume of crude extract (ml), L: loading sample.
Fig.4 shows an electrophoresis image of Step elution on CM sepharose Fast Flow;
   wherein, M: molecular mark, L: loading sample, FT: flow-through peak, 10%, 20%, 40% were elution salt concentrations, respectively.
Fig.5 shows an electrophoresis image of groping impurity-washing conditions on CM sepharose Fast Flow;
   wherein, M: molecular mark, L: loading sample, FT: flow-through peak, 10%, 20%, 40% are salt concentrations for washing impurities, respectively, Elu: rLF elution peak.
Fig. 6 shows HPLC purity of the obtained purified rLF product (RP -HPLC);
   wherein, the purity is higher than 95%.

### Detailed Description of the Invention

The features and advantages of the present invention can be further understood through the following detailed description in conjunction with the accompanying drawings. Examples are provided to illustrate the method of the invention, and should not to be construed as limiting the invention in any way.

Unless otherwise stated, the equipment and apparatus used in the following examples were commercially available.

### [Example 1] Preparation of Crude Extract of Recombinant Human Lactoferrin (rLF)

The paddy rice containing recombinant human lactoferrin was hulled to obtain brown rice and ground to obtain rice powder with a fineness of 80-100 mesh. The rice powder was mixed with an extraction buffer at a ratio of 1:10 (w/v, kg/L), and extracted at room temperature for 1 hour. The components of the extraction buffer were: 25mM Tris-HCl, 20mM anhydrous sodium acetate (NaAC), 200mM NaCl, pH 6.5. The obtained mixture was subjected to pressure filtration using a filter cloth type plate-frame press filter, to obtain clear crude extract containing rLF.

### [Example 2] Selection of Chromatography Resin and Elution Conditions in Cation Exchange Chromatography

### Selection of chromatography resin in cation exchange chromatography

Since the rice seeds have higher content of pigment and polysaccharide, a cation resin can avoid the deposition of pigments on the chromatography media, thereby improving the availability of resin and ensure the purity of the samples. This example employed cation exchange resins with higher work flow rate as chromatography resin, including UNO Sphere S from Bio-Rad company and CM sepharose Fast Flow (CM sepharose FF) from GE company and so on. The crude extract obtained in Example 1 was used as a loading sample of cation exchange chromatography.

### 1. Cation exchange chromatography performed on CM sepharose Fast Flow

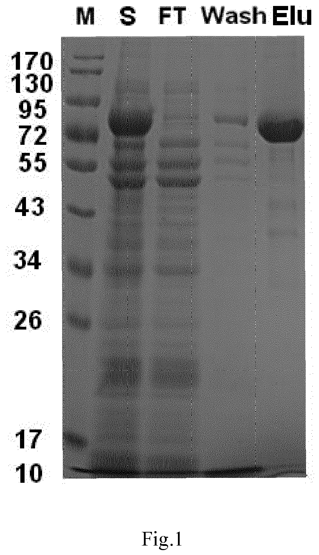

About 18ml of CM sepharose Fast Flow resin was packed onto an Econo-column 15/20 column, and the column was equilibrated with 200ml of buffer (25mM Tris-HCl, 20mM anhydrous sodium acetate, 200mM NaCl, pH6.5) at a flow rate of 150cm/h, until the pH value and the conductivity were constant to baseline. The sample had a conductivity of 22.5ms/cm and a pH of 6.5. After loading, the sample was eluted with a buffer (4mM NaH₂PO₄, 6mM Na₂HPO₄, 300mM NaCl, pH 7.5) at a flow rate of 150cm/h to elute impurities. After eluting the impurities, the lactoferrin was eluted with a buffer (4mM NaH₂PO₄, 6mM Na₂HPO₄, 600mM NaCl, pH 7.5) at a flow rate of 150cm/h, and the elution fractions were collected to obtain the rLF-containing fraction. The electrophoresis image is shown in Fig.1.

### 2. Cation exchange chromatography performed on UNO Sphere S

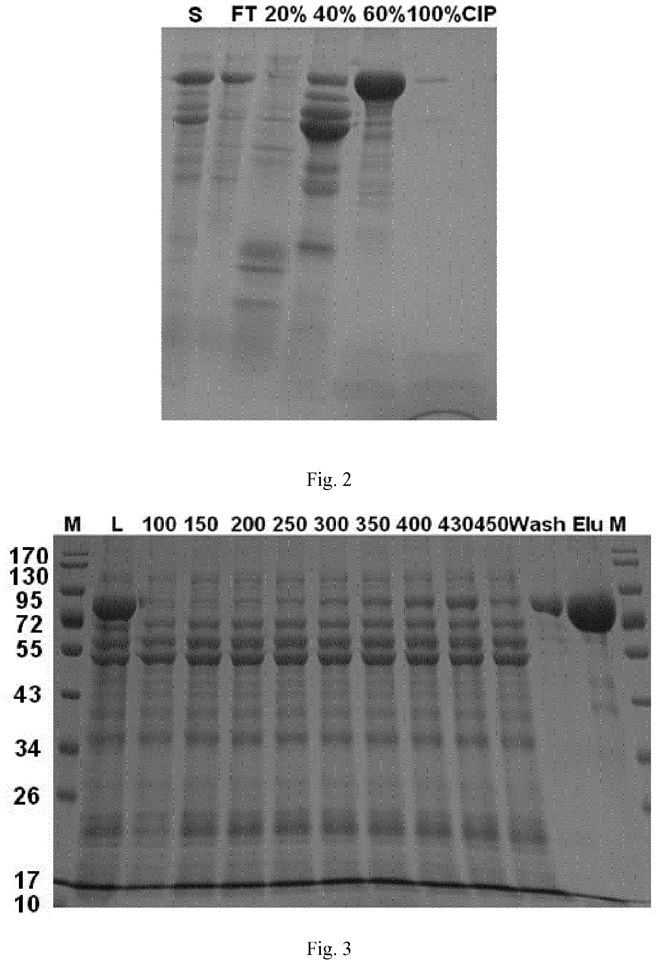

About 18ml of UNO Sphere S resin was packed onto an Econo-column 15/20 column, and the column was equilibrated with 200ml of buffer (25mM Tris-HCl, 20mM anhydrous sodium acetate, 200mM NaCl, pH 8.0) at a flow rate of 150cm/h, until the pH value and the conductivity were constant to baseline. The sample had a conductivity of 22.5ms/cm and a pH of 6.5. After loading, the sample was subjected to Step elution with a buffer containing 4mM NaH₂PO₄, 6mM Na₂HPO₄, pH 7.5 and containing 300mM NaCl, 600mM NaCl, 900mM NaCl, 1.5M NaCl respectively at a flow rate of 150cm/h, and the elution fractions were collected. The electrophoresis image is shown in Fig.2. In the figure, 20%, 40%, 60%, 100% are percentage ratios of 1.5M NaCl, respectively, corresponding to 300mM NaCl, 600mM NaCl, 900mM NaCl and 1.5M NaCl, respectively.

### 3. Determination of actual load capacity of CM sepharose Fast Flow

About 18ml of CM Sepharose Fast Flow was packed onto an Econo-column 15/20 column, and the column was equilibrated with 200ml of buffer (25mM Tris-HCl, 20mM anhydrous sodium acetate, 200mM NaCl, pH6.5) at a flow rate of 150cm/h, until the pH value and the conductivity were constant to baseline. The sample of Example 1 had a conductivity of 22.5 ms/cm and a pH of 6.5 and the total volume was 450ml. The sample was loaded onto CM sepharose Fast Flow at a flow rate of 150cm/h. Absorption value of UV280 during the sample loading was recorded until the absorption value beyond plateau by 10%. The sample volume was recorded and the actual load capacity per milliliter of CM sepharose Fast Flow under this flow rate was calculated; then the resin was regenerated. The electrophoresis image is shown in Fig.3.

It was found from the above experiments that under the same conditions of sample-loading, weak cation exchange resin CM Fast Flow exhibited higher resolution and separation efficiency, like a stronger anion exchange resin; meanwhile CM sepharose Fast Flow was developed earlier, and had mature process and excellent chemical stability, longer usage life and relative low cost. In view of the various factors, CM sepharose Fast Flow was used as a preferred chromatography resin for cation exchange chromatography.

### Optimization of elution conditions

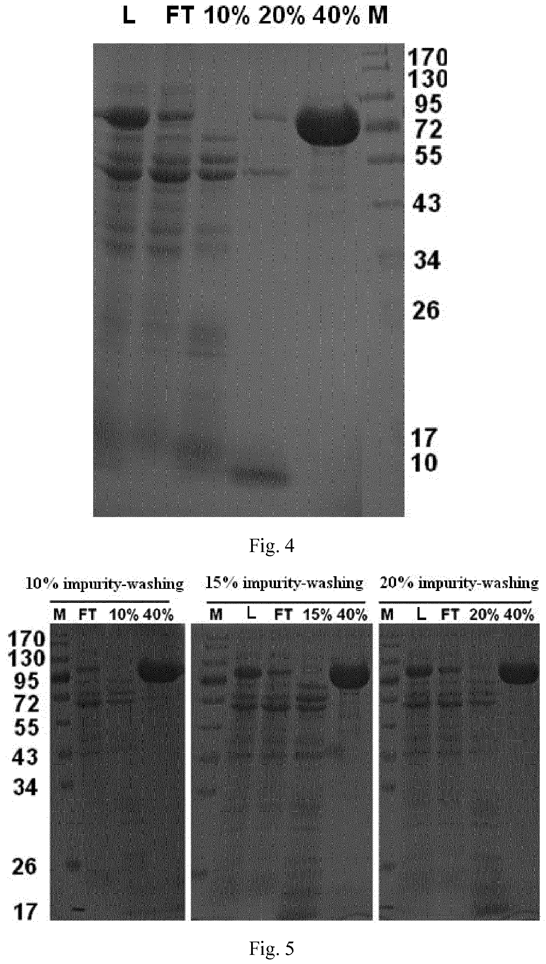

The rLF-containing extract was loaded onto a CM sepharose Fast Flow column and subjected to a gradient elution by adding a salt (Fig. 4). The results showed that, under the gradient of 10% 1.5M NaCl, obvious impurity bands were eluted, and no target protein was lost; under the gradient of 20% 1.5M NaCl, a small amount of impurity bands were eluted, and the loss of target protein was less; the target protein was eluted in concentrated form under the gradient of 40% 1.5M NaCl ; there was no elution peak under 60% or higher salt concentration, which meant that the target protein can be eluted completely under 40% 1.5M NaCl. Through further study on the elution concentration, it was found that when the salt concentration was less than 40%, the purity of eluted protein was not affected. The elution peak had serious peak tail and the recovery rate decreased. Finally, 40% 1.5M NaCl was selected as the determined elution condition.

### Optimization of impurity-washing conditions

Under the same equilibrium conditions and elution conditions, the impurity-washing conditions of 10%, 15% and 20% were compared, respectively (Fig.5). With the increase of salt concentration for impurity-washing, the loss of target protein was increased, but the impurity removal efficiency was significantly improved. Under the three conditions, there was no significant difference in the protein recovery rate. In order to ensure the purity of the target protein, 20% 1.5M NaCl was determined as the impurity-washing condition finally.

Taking purification efficiency and recovery rate into consideration, 4mM NaH₂PO₄, 6mM Na₂HPO₄, 300mM NaCl, pH 7.5 were used as the preferred impurity-washing conditions, and 4mM NaH₂PO₄, 6mM Na₂HPO₄, 600mM NaCl, pH 7.5 were used as the preferred elution conditions of the target protein.

### [Example 3] Determination of Regeneration Conditions of CM sepharose FF Resin after rLF Purification

After rLF purification through cation exchange, it was found that there was serious brown pigment deposition on the chromatography column during CIP (clean in place), which cannot be removed by conventional regeneration with water, 30% isopropyl alcohol, 70% ethanol, 0.01M HCl, 1M NaOH and 8M urea. It was known by analysis that the iron that was bound to rLF formed ferric hydroxide precipitate under strong alkaline. Since the resin cannot tolerate high concentration of acid, finally the deposited pigments were successfully removed through stronger ferric ion chelating property of sodium citrate under acid conditions.

During CM sepharose FF regeneration using sodium citrate, it was found that sodium citrate only chelated with iron on the column under low pH condition. Taking pH range that can be tolerated by resin into consideration, pH 3.0 was selected finally; under the normal usage of resin regeneration solution (about 5 times column volume), the concentration of citrate was determined. If ferric ions cannot be removed completely under this concentration, during the subsequent NaOH regeneration, brown pigment deposition would be present in the chromatography column. Otherwise, the chromatography column would show normal color. The experiment results are shown in the Table below:

| Concentration of sodium citrate | Color of chromatography column | Usage |
|---|---|---|
| 10mM | brown pigment deposition | 100ml |
| 20mM | normal | 100ml |
| 50mM | normal | 100ml |
| 100mM | normal | 100ml |

The results showed that: 100ml of 20mM sodium citrate can chelate with ferric ions in the chromatography column completely. The concentration of sodium citrate was thus determined.

To sum up, according to the particularity of the target protein, the regeneration process of CM Sepharose Fast Flow was changed from washing with normal high salt followed by NaOH into the following steps: removing rLF that was not eluted using high salt (2M NaCl, pH 7.0), then removing iron in the residual rLF using acid sodium citrate (20mM sodium citrate, pH 3.0), then performing normal cleaning procedure using 1M NaOH.

The chromatography column that was packed with 18ml of CM sepharose FF resin for cation exchange chromatography used in Example 1 was first washed with 100ml of high salt solution (2M NaCl, pH 7.0) at a flow rate of 150cm/h until UV280 had no obvious change; then washed with 100ml of buffer solution (20mM sodium citrate, pH 3.0) at a flow rate of 150cm/h until pH decreased below 4.0; then the chromatography column was back-washed with 100ml of 0.5M NaOH solution at a flow rate of 50cm/h; and then washed with 200ml of ultra pure water until pH was less than 10.0; finally, the resin was stored in 20% ethanol solution for a long time.

### [Example 4] Separation and Purification of recombinant human lactoferrin and regeneration of chromatography resin

### One-step purification performed by cation exchange

About 150ml of CM sepharose Fast Flow resin was packed onto an XK50 column, and the column was equilibrated with 2000ml of buffer (25mM Tris-HCl, 20mM anhydrous sodium acetate, 200mM NaCl, pH 6.5) at a flow rate of 150cm/h, until the pH value and the conductivity were constant to baseline. The crude extract obtained in Example 1 was used as a sample. The sample had a conductivity of 22.5ms/cm and a pH of 6.46. After loading, the sample was eluted with a buffer (4mM NaH₂PO₄, 6mM Na₂HPO₄, 300mM NaCl, pH 7.5) at a flow rate of 150cm/h to elute impurities and collect impurity-washing fraction; then the sample was eluted with a buffer (4mM NaH₂PO₄, 6mM Na₂HPO₄, 600mM NaCl, pH 7.5) at a flow rate of 150cm/h, and the elution fraction was collected to obtain rLF-containing fraction.

Further, the resin was regenerated by the following steps: it was first washed with 500ml of high salt solution (2M NaCl, pH 7.0) at a flow rate of 150cm/h until UV280 had no obvious change; then washed with 500ml of buffer solution (20mM sodium citrate, pH 3.0) at a flow rate of 150cm/h until pH decreased below 4.0; then the chromatography column was back-washed with 500ml of 0.5M NaOH solution at a flow rate of 50cm/h; and then washed with 1500ml of ultra pure water until pH was less than 10.0; finally, the resin was stored in 20% ethanol solution for a long time.

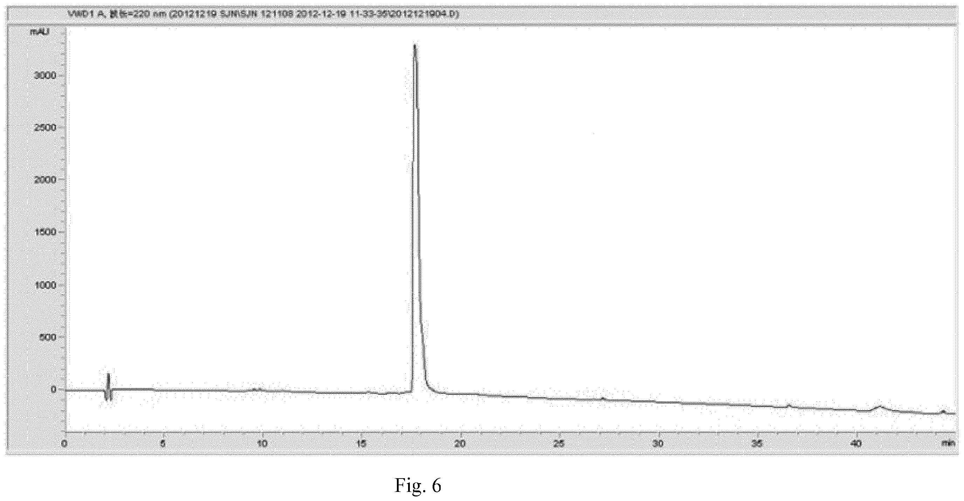

The rLF obtained from separation and purification was determined by HPLC. It showed that the HPLC purity of rLF was 98%, as shown in Fig.6; and the yield of rLF could reach 27.24±0.77%, corresponding to 2.25g rLF per kilogram brown rice, particularly as shown in the table below.

| Purification step | Total volume (ml) | Total protein content (mg) | Total lactoferrin (mg) | Total recovery of protein (%) | total recovery of LF (%) |
|---|---|---|---|---|---|
| rLF crude extract | 1750 | 1639 | 820 | 100 | 100 |
| rLF purified extract | 222 | 450 | 427 | 27.5 | 52.1 |

## Claims

1. A chromatography method for separating and purifying recombinant human lactoferrin from transgenic rice seeds, sequentially comprises the following steps of:
1) extracting recombinant human lactoferrin from transgenic rice seeds, to obtain crude extract containing recombinant human lactoferrin,
2) subjecting the crude extract containing recombinant human lactoferrin to cation exchange chromatography to perform primary purification, to obtain an elution fraction containing recombinant human lactoferrin; wherein:
the cation exchange chromatography is performed on a weak cation exchange resin CM sepharose FF , the chromatography step comprises:
2A) equilibrating the chromatography column with 5-15 times column volume of buffer containing 10-25mM Tris, 10-25mM NaAC, 0-250mM NaCl, pH 6.0-7.5, at a flow rate of 50-200cm/h;
2B) using the crude extract of step 1) as a loading sample, wherein the sample having a conductance of 2-25ms/cm and a pH of 6.0-7.5; eluting impurities with an impurity-washing buffer, the impurity-washing buffer containing 3-10mM NaH₂PO₄, 3-10mM Na₂HPO₄, 200-300mM NaCl, pH6.5-8.0, and the elution flow rate being 50-200cm/h;
2C) eluting the sample with a washing buffer containing 3-10mM NaH₂PO₄, 3-10mM Na₂HPO₄, 400-650mM NaCl, pH 6.5-8.0 at a flow rate of 50-200cm/h, to obtain an elution fraction;
3) collecting the elution fraction of step 2), to obtain the solution containing recombinant human lactoferrin.

2. The chromatography method of claim 1, wherein it further comprises the following chromatography resin regeneration steps of:
a) washing the column with 3-10 times column volume of high salt solution containing 1-2M NaCl, pH 6.5-10.0 at a flow rate of 50-200cm/h until UV280 has no obvious change;
b) washing the column with 3-10 times column volume of buffer containing 5-25mM sodium citrate, pH 2.0-4.0 at a flow rate of 50-200cm/h;
c) washing the column with 2-5 times column volume of 0.5-1.0M NaOH solution at a flow rate of 30-100cm/h;
d) washing the column with 8-15 times column volume of ultrapure water to a pH of less than 10.0, and keeping the resin in 20% ethanol solution.

3. The chromatography method of claim 1, wherein the extraction of recombinant human lactoferrin of step 1) comprises the following steps of:
(1) using transgenic rice seeds containing recombinant human lactoferrin as raw material, hulling the rice grains into brown rice and grinding it into rice powder with a fineness of 80-100 mesh;
(2) mixing the rice powder and an extraction buffer at a weight/volume ratio of 1:5-1:10 at room temperature for 0.5-2h, to obtain a mixture; the extraction buffer containing 10-25mM Tris, 10-25mM NaAC, 0-250mM NaCl, pH 6.0-7.5;
(3) filtrating the mixture, to obtain crude extract containing recombinant human lactoferrin.

4. The chromatography method of claim 1, wherein the chromatography step of step 2) comprises:
2a) equilibrating the chromatography column with 10 times column volume of equilibrium buffer containing 25mM Tris, 25mM NaAC, 200mM NaCl, pH 6.5, at a flow rate of 150cm/h;
2b) using the crude extract of step 1) as a loading sample, wherein the sample having a conductance of 22.5ms/cm and a pH of 6.5; washing the impurity with an washing buffer, the washing buffer containing 4mM NaH₂PO₄, 6mM Na₂HPO₄, 300mM NaCl, pH 7.5 at a flow rate of 150cm/h;
2c) eluting the sample with an elution buffer, the elution buffer containing 4mM NaH₂PO₄, 6mM Na₂HPO₄, 600mM NaCl, pH 7.5 at a flow rate of 150cm/h, to collect the elution fraction containing recombinant human lactoferrin.

5. The chromatography method of claim 1, wherein the chromatography resin regeneration step comprises:
a1) washing the column with 5 times column volume of high salt solution containing 2M NaCl, pH 7.5 at a flow rate of 150cm/h until UV280 has no obvious change;
b1) washing the column with 5 times column volume of buffer containing 20mM sodium citrate, pH 3.0 at a flow rate of 50-200cm/h;
c1) washing the column with 3 times column volume of 0.5M NaOH solution at a flow rate of 50cm/h;
d1) washing the column with 10 times column volume of ultrapure water to a pH of less than 10.0, and keeping the resin in 20% ethanol solution.

6. The chromatography method of claim 3, wherein the extraction of recombinant human lactoferrin comprises the following steps of:
(1a) using transgenic rice seeds containing recombinant human lactoferrin as raw material, hulling the rice grains into brown rice and grinding it into rice powder with a fineness of 80-100 mesh;
(2a) mixing the rice powder and an extraction buffer at a weight/volume ratio of 1:10 at room temperature for 1h, to obtain a mixture; the extraction buffer containing 25mM Tris, 20mM NaAC, 200mM NaCl, pH 6.5;
(3a) filtrating the mixture, to obtain crude extract containing recombinant human lactoferrin.

## Patentansprüche

1. Chromatographieverfahren zur Trennung und Reinigung von rekombinantem menschlichem Lactoferrin aus transgenen Reissamen, sequentiell die folgenden Schritte umfassend:
1) Extrahieren von rekombinantem menschlichem Lactoferrin aus transgenen Reissamen, um einen Rohextrakt zu erhalten, der rekombinantes menschliches Lactoferrin enthält,
2) Unterziehen des Rohextrakts, der rekombinantes menschliches Lactoferrin enthält, einer Kationenaustauschchromatographie, um eine Primärreinigung durchzuführen, um eine Elutionsfraktion zu erhalten, die rekombinantes menschliches Lactoferrin enthält; wobei:
die Kationenaustauschchromatographie an einem schwachen Kationenaustauscherharz CM-Sepharose FF durchgeführt wird, wobei der Chromatographieschritt Folgendes umfasst:
2A) Äquilibrieren der Chromatographiesäule mit dem 5-15-fachen Säulenvolumen eines Puffers, enthaltend 10-25 mM Tris, 10-25 mM NaAC, 0-250 mM NaCl, pH-Wert 6,0-7,5, bei einer Flussrate von 50-200 cm/h;
2B) Verwenden des Rohextrakts aus Schritt 1) als Beladungsprobe, wobei die Probe eine Leitfähigkeit von 2-25 ms/cm und einen pH-Wert von 6,0-7,5 aufweist; Eluieren von Verunreinigungen mit einem Verunreinigungs-Waschpuffer, wobei der Verunreinigungs-Waschpuffer 3-10 mM NaH₂PO₄, 3-10 mM Na₂HPO₄, 200-300 mM NaCl, pH-Wert 6,5-8,0 enthält, und wobei die Elutionsflussrate 50-200 cm/h beträgt;
2C) Eluieren der Probe mit einem Waschpuffer, enthaltend 3-10 mM NaH₂PO₄, 3-10 mM Na₂HPO₄, 400-650 mM NaCl, pH-Wert 6,5-8,0, bei einer Flussrate von 50-200 cm/h, um eine Elutionsfraktion zu erhalten;
3) Sammeln der Elutionsfraktion aus Schritt 2), um die rekombinantes menschliches Lactoferrin enthaltende Lösung zu erhalten.

2. Chromatographieverfahren nach Anspruch 1, wobei es ferner die folgenden Chromatographieharz-Regenerationsschritte umfasst:
a) Waschen der Säule mit dem 3-10-fachen Säulenvolumen einer Lösung mit hohem Salzgehalt, enthaltend 1-2 M NaCl, pH-Wert 6,5-10,0, bei einer Flussrate von 50-200 cm/h, bis UV280 keine offensichtliche Veränderung zeigt;
b) Waschen der Säule mit dem 3-10-fachen Säulenvolumen eines Puffers, enthaltend 5-25 mM Natriumcitrat, pH-Wert 2,0-4,0, bei einer Flussrate von 50-200 cm/h;
c) Waschen der Säule mit dem 2,5-fachen Säulenvolumen einer 0,5-1,0 M NaOH-Lösung, bei einer Flussrate von 30-100 cm/h;
d) Waschen der Säule mit dem 8-15-fachen Säulenvolumen von ultrareinem Wasser bis zu einem pH-Wert von unter 10,0, und Aufbewahren des Harzes in 20 % Ethanollösung.

3. Chromatographieverfahren nach Anspruch 1, wobei die Extraktion von rekombinantem menschlichem Lactoferrin aus Schritt 1) die folgenden Schritte umfasst:
(1) Verwenden von transgenen Reissamen, die rekombinantes menschliches Lactoferrin enthalten, als Rohmaterial, Enthülsen der Reiskörner zu braunem Reis und Zermahlen des letzteren zu Reispulver mit einer Feinheit von 80-100 Mesh;
(2) Mischen des Reispulvers und eines Extraktionspuffers bei einem Gewicht/Volumen-Verhältnis von 1:5-1:10 bei Raumtemperatur für 0,5-2 h, um ein Gemisch zu erhalten; wobei der Extraktionspuffer 10-25 mM Tris, 10-25 mM NaAC, 0-250 mM NaCl, pH-Wert 6,0-7,5 enthält;
(3) Filtrieren des Gemischs, um Rohextrakt zu erhalten, der rekombinantes menschliches Lactoferrin enthält.

4. Chromatographieverfahren nach Anspruch 1, wobei der Chromatographieschritt nach Schritt 2) Folgendes umfasst:
2a) Äquilibrieren der Chromatographiesäule mit dem 10-fachen Säulenvolumen eines Äquilibrierpuffers, enthaltend 25 mM Tris, 25 mM NaAC, 200 mM NaCl, pH-Wert 6,5, bei einer Flussrate von 150 cm/h;
2b) Verwenden des Rohextrakts aus Schritt 1) als eine Beladungsprobe, wobei die Probe eine Leitfähigkeit von 22,5 ms/cm und einen pH-Wert von 6,5 aufweist; Waschen der Verunreinigung mit einem Waschpuffer, wobei der Waschpuffer 4 mM NaH₂PO₄, 6 mM Na₂HPO₄, 300 mM NaCl, pH-Wert 7,5 enthält, bei einer Flussrate von 150 cm/h;
2c) Eluieren der Probe mit einem Elutionspuffer, wobei der Elutionspuffer 4 mM NaH₂PO₄, 6 mM Na₂NPO₄, 600 mM NaCl, pH-Wert 7,5 enthält, bei einer Flussrate von 150 cm/h, um die Elutionsfraktion zu sammeln, die rekombinantes menschliches Lactoferrin enthält.

5. Chromatographieverfahren nach Anspruch 1, wobei der Chromatographieharz-Regenerationsschritt Folgendes umfasst:
a1) Waschen der Säule mit dem 5-fachen Säulenvolumen einer Lösung mit hohem Salzgehalt, enthaltend 2 M NaCl, pH-Wert 7,5, bei einer Flussrate von 150 cm/h, bis UV280 keine offensichtliche Veränderung zeigt;
b1) Waschen der Säule mit dem 5-fachen Säulenvolumen eines Puffers, enthaltend 20 mM Natriumcitrat, pH-Wert 3,0, bei einer Flussrate von 50-200 cm/h;
c1) Waschen der Säule mit dem 3-fachen Säulenvolumen einer 0,5 M NaOH-Lösung, bei einer Flussrate von 50 cm/h;
d1) Waschen der Säule mit dem 10-fachen Säulenvolumen von ultrareinem Wasser bis zu einem pH-Wert von unter 10,0, und Aufbewahren des Harzes in 20 % Ethanollösung.

6. Chromatographieverfahren nach Anspruch 3, wobei die Extraktion von rekombinantem menschlichem Lactoferrin die folgenden Schritte umfasst:
(1a) Verwenden von transgenen Reissamen, die rekombinantes menschliches Lactoferrin enthalten, als Rohmaterial, Enthülsen der Reiskörner zu braunem Reis und Zermahlen des letzteren zu Reispulver mit einer Feinheit von 80-100 Mesh;
(2) Mischen des Reispulvers und eines Extraktionspuffers bei einem Gewicht/Volumen-Verhältnis von 1:10 bei Raumtemperatur für 1 h, um ein Gemisch zu erhalten; wobei der Extraktionspuffer 25 mM Tris, 20 mM NaAC, 200 mM NaCl, pH-Wert 6,5 enthält;
(3a) Filtrieren des Gemischs, um Rohextrakt zu erhalten, der rekombinantes menschliches Lactoferrin enthält.

## Revendications

1. Méthode chromatographique servant à la séparation et à la purification de lactoferrine humaine recombinée à partir de grains de riz transgéniques, ladite méthode comprenant dans l'ordre les étapes suivantes consistant à :
1) extraire la lactoferrine humaine recombinée à partir de grains de riz transgéniques, afin d'obtenir un extrait brut contenant de la lactoferrine humaine recombinée,
2) soumettre l'extrait brut contenant de la lactoferrine humaine recombinée à une chromatographie à échange de cations pour réaliser une première purification, afin d'obtenir une fraction d'élution contenant la lactoferrine humaine recombinée ; dans laquelle :
la chromatographie à échange de cations est réalisée sur une résine de CM sépharose FF faible échangeuse de cations, l'étape de chromatographie consiste à :
2A) équilibrer la colonne de chromatographie avec 5 à 15 fois le volume de la colonne d'une solution tampon contenant de 10 à 25 mM de Tris, de 10 à 25 mM de NaAC, de 0 à 250 mM de NaCl, à un pH de 6,0 à 7,5, et à un débit de 50 à 200 cm/h ;
2B) utiliser l'extrait brut de l'étape 1) comme échantillon de charge, dans laquelle l'échantillon présente une conductance de 2 à 25 ms/cm et un pH de 6,0 à 7,5 ; éluer les impuretés à l'aide d'une solution tampon permettant de laver les impuretés, la solution tampon permettant de laver les impuretés contenant de 3 à 10 mM de NaH₂PO₄, de 3 à 10 mM de Na₂HPO₄, de 200 à 300 mM de NaCl, à un pH de 6,5 à 8,0, et le débit d'élution étant de 50 à 200 cm/h ;
2C) éluer l'échantillon avec une solution tampon de lavage contenant de 3 à 10 mM de NaH₂PO₄, de 3 à 10 mM de Na₂HPO₄, de 400 à 650 mM de NaCl, à un pH de 6,5 à 8,0 et à un débit de 50 à 200 cm/h, afin d'obtenir une fraction d'élution ;
3) récupérer la fraction d'élution de l'étape 2), afin d'obtenir la solution contenant de la lactoferrine humaine recombinée.

2. Méthode chromatographique selon la revendication 1, comprenant en outre les étapes suivantes de régénération de la résine de chromatographie consistant à :
a) laver la colonne avec 3 à 10 fois le volume de la colonne d'une solution à teneur élevée en sels contenant de 1 à 2 M de NaCl, à un pH de 6,5 à 10,0 et à un débit de 50 à 200 cm/h jusqu'à ce qu'aucun changement évident ne soit observé à UV280 ;
b) laver la colonne avec 3 à 10 fois le volume de la colonne d'une solution tampon contenant de 5 à 25 mM de citrate de sodium, à un pH de 2,0 à 4,0 et à un débit de 50 à 200 cm/h ;
c) laver la colonne avec 2 à 5 fois le volume de la colonne d'une solution de 0,5 à 1,0 M de NaOH à un débit de 30 à 100 cm/h ;
d) laver la colonne avec 8 à 15 fois le volume de la colonne d'une eau ultrapure à un pH inférieur à 10,0, et maintenir la résine dans une solution à 20 % d'éthanol.

3. Méthode chromatographique selon la revendication 1, dans laquelle l'extraction de la lactoferrine humaine recombinée de l'étape 1) comprend les étapes suivantes consistant à :
(1) utiliser des grains de riz transgéniques contenant de la lactoferrine humaine recombinée comme matière première, écosser les grains de riz jusqu'à obtenir du riz brun et le broyer jusqu'à obtenir une poudre de riz présentant une finesse de 80 à 100 mesh ;
(2) mélanger la poudre de riz et une solution tampon d'extraction à un rapport poids/volume de 1:5 à 1:10 à température ambiante pendant 0,5 à 2 h, afin d'obtenir un mélange ; la solution tampon d'extraction contenant de 10 à 25 mM de Tris, de 10 à 25 mM de NaAC, de 0 à 250 mM de NaCl, à un pH de 6,0 à 7,5 ;
(3) filtrer le mélange, afin d'obtenir un extrait brut contenant de la lactoferrine humaine recombinée.

4. Méthode chromatographique selon la revendication 1, dans laquelle l'étape de chromatographie de l'étape 2) comprend :
2a) l'équilibrage de la colonne de chromatographie avec 10 fois le volume de la colonne d'une solution tampon d'équilibre contenant 25 mM de Tris, 25 mM de NaAC, 200 mM de NaCl, à un pH de 6,5, et à un débit de 150 cm/h ;
2b) l'utilisation de l'extrait brut de l'étape 1) comme échantillon de charge, dans laquelle l'échantillon présente une conductance de 22,5 ms/cm et un pH de 6,5 ; laver les impuretés avec une solution tampon de lavage, la solution tampon de lavage contenant 4 mM de NaH₂PO₄, 6 mM de Na₂HPO₄, 300 mM de NaCl, à un pH de 7,5 et à un débit de 150 cm/h ;
2c) l'élution de l'échantillon avec une solution tampon d'élution, la solution tampon d'élution contenant 4 mM de NaH₂PO₄, 6 mM de Na₂HPO₄, 600 mM de NaCl, à un pH de 7,5 et à un débit de 150 cm/h, afin de récupérer la fraction d'élution contenant de la lactoferrine humaine recombinée.

5. Méthode chromatographique selon la revendication 1, dans laquelle l'étape de régénération de la résine de chromatographie comprend :
a1) le lavage de la colonne avec 5 fois le volume de la colonne d'une solution à teneur élevée en sels contenant 2 M de NaCl, à un pH de 7,5 et à un débit de 150 cm/h jusqu'à ce qu'aucun changement évident ne soit observé à UV280 ;
b1) le lavage de la colonne avec 5 fois le volume de la colonne d'une solution tampon contenant 20 mM de citrate de sodium, à un pH de 3,0 et à un débit de 50 à 200 cm/h ;
cl) le lavage de la colonne avec 3 fois le volume de la colonne d'une solution à 0,5 M de NaOH à un débit de 50 cm/h ;
d1) laver la colonne avec 10 fois le volume de la colonne d'une eau ultrapure à un pH inférieur à 10,0, et maintenir la résine dans une solution à 20 % d'éthanol.

6. Méthode chromatographique selon la revendication 3, dans laquelle l'extraction de la lactoferrine humaine recombinée comprend les étapes suivantes consistant à :
(1a) utiliser des grains de riz transgéniques contenant de la lactoferrine humaine recombinée comme matière première, écosser les grains de riz jusqu'à obtenir du riz brun et le broyer jusqu'à obtenir une poudre de riz présentant une finesse de 80 à 100 mesh ;
(2a) mélanger la poudre de riz et une solution tampon d'extraction à un rapport poids/volume de 1:10 à température ambiante pendant 1 h, afin d'obtenir un mélange ; la solution tampon d'extraction contenant 25 mM de Tris, 20 mM de NaAC, 200 mM de NaCl, à un pH de 6,5 ;
(3a) filtrer le mélange, afin d'obtenir un extrait brut contenant la lactoferrine humaine recombinée.
